# EUROPEAN PATENT SPECIFICATION

(11) **EP 0 540 508 B1**
(45) Date of publication and mention of the grant of the patent: **06.03.1996**
(21) Application number: 93200242.1
(22) Date of filing: 31.07.1986
(51) Int. Cl.: C08G 73/10, C08G 59/40, C08L 63/00, C07D 403/04

(54) **Imide hardeners and their preparation**
Imide-Härter und ihre Herstellung
Imides durcisseurs et leur préparation

(30) Priority: 31.07.1985 JP 170052/85; 12.03.1986 JP 53922/86
(43) Date of publication of application: 05.05.1993
(62) Divisional of application: 86305901.0
(73) Proprietor: SUMITOMO CHEMICAL COMPANY, LIMITED, Osaka (JP)
(72) Inventor: Saito, Yasuhisa, Toyonaka, Osaka-fu (JP); Kanagawa, Shuichi, Tukuba, Ibaragi-ken (JP); Watanabe, Katsuya, Takatsuki, Osaka-fu (JP); Kamio, Kunimasa, Toride, Ibaragi-ken (JP)
(74) Representative: Geering, Keith Edwin

(56) References cited:
- FR-A- 1 476 000
- US-A- 3 410 876

## Description

The present invention relates to imide compounds and to epoxy resin compositions which contain imide compound and epoxy resin and are suitable for lamination and molding.

Hitherto, for sealing laminates and semiconductor elements such as IC and LSI. used in apparatus for industry and trade, epoxy resins have been used.

With laminates, however, there is a large change in dimension perpendicular to the substrate because of the low thermal resistance of the hardened epoxy resin, giving problems such as lowering in through-hole reliability and smear. With sealing material for IC and LSI. there is also the problem that when parts are connected to circuits by soldering, the laminates are cracked by the heat of the solder because of the large thermal expansion of the epoxy material.

Aromatic imide compounds have been used as hardener to improve the thermal resistance of such hardened epoxy product.

Generally, aromatic imide compounds are produced from aromatic tetracarboxylic acid anhydrides and aromatic diamines. The well-known representative aromatic tetracarboxylic acid anhydrides include pyromellitic acid anhydride, benzophenonetetracarboxylic acid anhydride. The aromatic imide compounds obtained from these acid anhydrides, however, have the problems that, generally, they are of very low solubility in low-boiling organic solvents, so that for dissolving them it is necessary to use special high-boiling solvents such as dimethylformamide, dimethylacetamide, N-methylpyrrolidone, dimethyl sulfoxide and cresol; and that they also have poor compatibility with epoxy resins, phenolic resins, amines and acid anhydrides, so that it is difficult to attain improvements in performances when using them with epoxy resins.

US-A-3 410 876 teaches a method for making dianhydrides from vinyl benzenes and maleic anhydride and discloses the reaction of 3,4-dicarboxy-1,2,3,4-tetrahydro-1-naphthalenesuccinic anhydride (**TDA**) with m-phenylene diamine to yield a polyimide (see example 4). Said polyimide does not contain an alkyl group R₁ in the 1-position of the tetrahydronaphthalene ring system.

We have found that imide compounds having a structural unit represented by the general formula 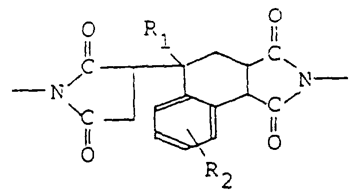 (wherein R₁ represents a C₁-C₁₀ alkyl group, and R₂ represents a hydrogen atom, a C₁-C₂₀ alkyl or alkoxy group or a hydroxyl group) in the molecule are of good solubility and compatibility, and that the foregoing problems such as low thermal resistance, large changes in dimension and cracking of laminates by heat can be solved by using these imide compounds and epoxy resins together.

The present invention provides an imide compound of formula (I), 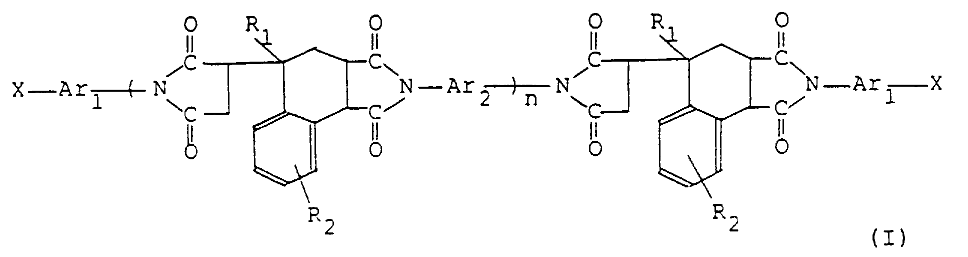 wherein the X's are selected from hydroxyl and amino groups; the Ar₁'s, and the or each Ar₂ if present, are selected independently from aromatic residues; the or each R₁ is selected independently from C₁-C₁₀ alkyl groups; the or each R₂ is selected independently from a hydrogen atom, a hydroxyl group and C₁-C₂₀ alkyl and alkoxy groups; and n is zero or an integer of up to 30. The invention also provides an epoxy resin composition containing imide compound (B) and epoxy resin (A) as essential components.

Referring to Ar₁ and Ar₂ in more detail, they are independently mononuclear or polynuclear divalent aromatic residues of which the aromatic ring may or may not be substituted, e.g. with a lower alkyl group, a halogen atom and a lower alkoxy group. Specifically, both Ar₁ and Ar₂ are the residues of aromatic amines. More specifically, the Ar₁'s are the residues of aromatic monoamines or diamines, and the Ar₂'s are the residues of aromatic diamines.

Of the aromatic amines, the aromatic diamines include for example: 4,4'-diaminodiphenylmethane, 3,3'-diaminodiphenylmethane, 4,4'diaminodiphenyl ether, 3,4'-diaminodiphenyl ether,4,4'-diaminodiphenylpropane,4,4'-diaminodiphenyl sulfone,3,3'-diaminodiphenyl sulfone,2,4-tolylenediamine,2,6-tolylenediamine,m-phenylenediamine, p-phenylenediamine, benzidine, 4,4'-diaminodiphenyl sulfide, 3,3'-dichloro-4,4'-diaminodiphenyl sulfone, 3,3'-dichloro-4,4'-diaminodiphenylpropane, 3,3'-dimethyl-4,4'-diaminodiphenylmethane, 3,3'-dimethoxy-4,4'-diaminobiphenyl, 3,3'-dimethyl-4,4'-diaminobiphenyl, 1,3-bis(4-aminophenoxy)benzene, 1,3-bis(3-aminophenoxy)benzene, 1,4-bis(4-aminophenoxy)benzene, 2,2-bis(4-aminophenoxyphenyl)propane, 4,4'-bis(4-aminophenoxy)diphenyl sulfone, 4,4'-bis(3-aminophenoxy)diphenyl sulfone, 9,9'-bis(4-aminophenyl)anthracene, 9,9'-bis(4-aminophenyl)fluorene, 3,3'-dicarboxy-4,4'-diaminodiphenylmethane, 2,4-diaminoanisole, bis(3-aminophenyl)methylphosphine oxide, 3,3'-diaminobenzophenone, o-toluidine sulfone, 4,4'-methylene-bis-o-chloroaniline, tetrachlorodiaminodiphenylmethane, m-xylylenediamine, p-xylylenediamine, 4,4'-diaminostilbene, 5-amino-1-(4'-aminophenyl)-1,3,3-trimethylindane, 6-amino-1-(4'-aminophenyl)-1,3,3-trimethylindane, 5-amino-6-methyl-1-(3'-amino-4'-methylphenyl)-1,3,3-trimethylindane, 7-amino-6-methyl-1-(3'-amino-4'-methylphenyl)-1,3,3-trimethylindane and 6-amino-5-methyl-1-(4'-amino-3'-methylphenyl)-1,3,3-trimethylindane, 6-amino-7-methyl-1-(4'-amino-3'-methylphenyl)-1,3,3-trimethylindane. These compounds may be used alone or in combination.

The aromatic monoamines include for example : o-aminophenol, m-aminophenol, p-aminophenol, 6-amino-m-cresol, 4-amino-m-cresol, 2,2-(4-hydroxyphenyl-4-aminophenyl)propane, 2,2-(4-hydroxyphenyl-2'-methyl-4'-aminophenyl)propane, 2,2-(3-methyl-4-hydroxyphenyl-4'-aminophenyl)propane, 8-amino-1-naphthol, 8-amino-2-naphthol, 5-amino-1-naphthol and 4-amino-2-methyl-1-naphthol. These compounds pounds may be used alone or in combination.

R₁ and R₂ are as defined above.

When substituent X in formula (I) is an amino group, imide compound (I) can be synthesized by reacting an excess of

aromatic diamine with compound of formula (III), 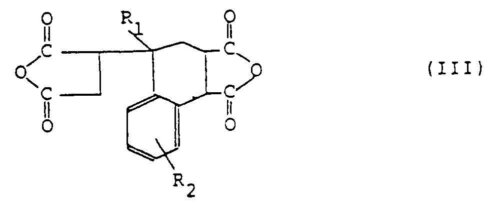 wherein R₁ and R₂ are as defined above,
according to the common imidation technique. When the substituent X in formula (I) is a hydroxyl group,

imide compound (I) can be synthesized by adding aromatic monoamine having a hydroxyl group and aromatic diamine to compound (III) so that the molar ratio of aromatic diamine to compound (III) is n to (n+1) wherein n is as defined above and

the molar ratio of aromatic monoamine to compound (III) is 2 to (n+1), and carrying out reaction according to the common imidation technique.

Compound (III) can be obtained by reacting compound of formula (IV), 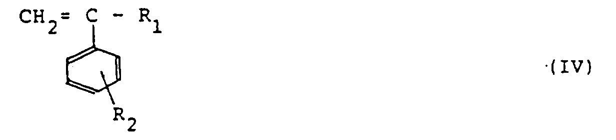 wherein R⁵ and R⁶ are as defined above,
with maleic anhydride at a molar ratio of compound (IV) to maleic anhydride of 1 to 2 in the absence of radical polymerization catalyst and in the presence or absence of radical polymerization inhibitor. The compounds (IV) include styrene, α-methylstyrene, α,p-dimethylstyrene, α,m-dimethylstyrene, isopropylstyrene, p-isopropenylphenol, m-isopropenylphenol, 1-methoxy-3-isopropenylbenzene and 1-methoxy-4-isopropenylbenzene. These compounds may be used alone or in combination.

The imide compounds (I) are soluble in high concentrations in low-boiling solvents such as acetone, methyl ethyl ketone, methyl isobutyl ketone, methyl cellosolve, ethyl cellosolve^{R}, methylene chloride, chloroform, etc. Also, they are of good compatibility with epoxy resins, phenolic resins, amines, acid anhydrides, etc.

The imide compounds (I) of the present invention are thermosettable by mixing with epoxy resins, etc. The hardened product obtained has good thermal resistance, mechanical characteristics; solvent resistance, etc.

The epoxy resin which can be used in combination with the imide of the present invention is for example a compound having two or more epoxy groups in the molecule. Examples of the epoxy resin include glycidyl ether compounds derived from dihydric or polyhydric phenols [e.g. bisphenol A, bisphenol F, hydroquinone, resorcinol, phloroglucinol, tris(4-hydroxyphenyl)methane, 1,1,2,2-tetrakis(4-hydroxyphenyl)ethane] or halogenated bisphenols (e.g. tetrabromobisphenol A); novolak type epoxy resins derived from novolak resins which are reaction products of phenols (e.g. phenol, o-cresol) with formaldehyde; amine type epoxy resins derived from aniline, p-aminophenol, m-aminophenol, 4-amino-m-cresol, 6-amino-m-cresol, 4,4'-diaminodiphenylmethane, 3,3'-diaminodiphenylmethane, 4,4'-diaminodiphenyl ether, 3,4'-daminodiphenyl ether, 1,4-bis(4-aminophenoxy)benzene, 1,4-bis(3-aminophenoxy)benzene, 1,3-bis(4-aminophenoxy)benzene, 1,3-bis(3-aminophenoxy)benzene, 2,2-bis(4-aminophenoxyphenyl)propane, p-phenylenediamine, m-phenylenediamine, 2,4-tolylenediamine, 2,6-tolylenediamine, p-xylylenediamine, m-xylylenediamine, 1,4-cyclohexane-bis(methylamine), 1,3-cyclohexane-bis (methylamine) and 5-amino-1-(4'-aminophenyl)-1,3,3-trimethylindane, 6-amino-1-(4'-aminophenyl)-1,3,3-trimethylindane, glycidyl ester compounds derived from aromatic carboxylic acids (e.g. p-oxybenzoic acid, m-oxybenzoic acid, terephthalic acid, isophthalic acid); hydantoin type epoxy resins derived from 5,5-dimethylhydantoin; alicyclic epoxy resins such as 2,2'-bis(3,4-epoxycyclohexyl)propane, 2,2-bis[4-(2,3-epoxypropyl)cyclohexyl]propane, vinylcyclohexene dioxide, 3,4-epoxycyclohexylmethyl-3,4-epoxycyclohexanecarboxylate, etc.; and other compounds such as triglycidyl isocyanulate and 2,4,6-triglycidoxy-S-triazine. These epoxy resins may be used alone or in combination.

The epoxy resin composition contains epoxy resin and imide compound (I) of the present invention as essential components, and if necessary, may further contain well-known epoxy hardeners and hardening accelerators, fillers, flame retardants, reinforcing agents, surface-treating agents, pigments, etc.

The well-known epoxy hardeners include for example amine type hardeners such as the foregoing aromatic amines (e.g. xylylenediamine), aliphatic amines, etc.; polyphenols such as phenol novolak, cresol novolak, etc.; and acid anhydrides, dicyandiamide, hydrazides, etc.

Preferably the total amount of (B) and other hardeners is 0.8 to 1.2 gram equivalents per gram equivalent of (A), and besides the amount of (B) is 0.4 to 1.2 gram equivalents per gram equivalent of (A). The hardening accelerators include for example amines [e.g. benzyldimethylamine, 2,4,6-tris(dimethylaminomethyl)phenol, 1,8-diazabicycloundecene], imidazole compounds (e.g. 2-ethyl-4-methylimidazole) and boron trifluoride amine complexes. The fillers include silica, calcium carbonate, etc.; the flame retardants include aluminum hydroxide, antimony trioxide and red phosphorus ; and the reinforcing agents include fabrics comprising organic or inorganic fibers (e.g. glass fibers, polyester fibers, polyamide fibers, alumina fibers), non-woven fabrics, mats, papers and combinations thereof.

The epoxy resin compositions of the invention can give hardened products of higher thermal resistance than obtainable by the prior art, and are of high value industrially as material for lamination and molding.

The present invention is illustrated in more detail by the following Examples, but it is not limited to these Examples.

### Example 1

To a flask equipped with a stirrer, a thermometer and a cooling separator were added 29.7 g (0.15 mole) of 4,4'-diaminodiphenylmethane and 242 g of m-cresol, and after dissolving the diaminodiphenylmethane in the m-cresol, 48.5 g of xylene was added. After heating the resulting mixture to a temperature of 120°C, 31.4 g (0.1 mole) of 1-methyl-3,4-dicarboxy-1,2,3,4-tetrahydro-1-naphthalenesuccinic acid anhydride was added at this temperature, and after raising the temperature to 175°C, dehydration was continued at this temperature for 5 hours. After completion of the reaction, a hexane/isopropanol mixed solution was added to precipitate the reaction product. The product was then washed two times with the same mixed solution and dried under reduced pressure to obtain an imide compound. This imide compound had a melting point of 241°C and an amine equivalent of 634 g/eq.

### Example 2

Example 1 was repeated except that 24.4 g (0.2 mole) of 2,4-tolylenediamine was used in place of 29.7 g (0.15 mole) of 4,4'-diaminodiphenylmethane, to obtain an imide compound. This compound had a melting point of about 220°C and an amine equivalent of 353 g/eq.

### Example 3

Example 1 was repeated except that 24.0 g (0.22 mole) of m-aminophenol was used in place of 29.7 g (0.15 mole) of 4,4'-diaminodiphenylmethane, to obtain an imide compound. This compound had a melting point of about 210°C and a hydroxyl equivalent of 249 g/eq.

The imide compounds obtained in Examples 1 to 3 are soluble in solvents such as acetone, MEK, methylene chloride, methyl cellosolve^{R}, etc.

### Example 4

100 Grams of Sumi ® epoxy ELA-128 (bisphenol A type epoxy resin; epoxy equivalent, 187 g/eq; a product of Sumitomo Chemical Co.) and 163 g of the imide compound obtained in Example 1 were uniformly dissolved in 180 g of dimethylformamide. This solution was impregnated into glass cloth (WE 18K-BZ-2; a product of Nitto Boseki Co., Ltd.), which was then treated for 5 minutes in an oven kept at
180°C to obtain prepreg. Six pieces of prepreg and a copper foil (a product of Furukawa Circuit Foil Co.; TAI treatment, thickness, 35µ) were placed one upon another and press-molded at
180°C for 5 hours under a pressure of 50 kg/cm to obtain a copper-lined laminate 1mm thick. The physical properties of this laminate were measured according to JIS C 6481 to obtain the result shown in Table 1.

### Example 5

Example 4 was repeated except that 90 g of the imide compound obtained in Example 2 was used in place of the imide compound obtained in Example 1, and that the amount of dimethylformamide was changed from 180 g to 130 g, to obtain a laminate. The physical properties of this laminate are shown in Table 1.

### Comparative example 1

240 Grams of Sumi ® epoxy ESA-011 (bisphenol A type epoxy resin; epoxy equivalent, 489 g/eq; a product of Sumitomo Chemical Co.), 20 g of Sumi ® epoxy ESCN-220 (o-cresol novolak type epoxy resin; epoxy equivalent, 210 g/eq; a product of Sumitomo Chemical Co.), 9 g of dicyandiamide and 1 g of 2-phenyl-4-methyl-5-hydroxymethylimidazole were dissolved in a mixed solvent of 40 g of dimethylformamide, 60 g of ethylene glycol monomethyl ether and 60 g of methyl ethyl ketone. As in Example 4, the resulting solution was impregnated into glass cloth, which was then treated for 5 minutes in an oven kept at 160°C to prepare prepreg. This prepreg was press-molded under the same conditions as in Example 4 to prepare a laminate. The physical properties of the laminate are shown in Table 1.

**Table 1**

| Example | | Example 4 | Example 5 | Comparative example 1 |
|---|---|---|---|---|
| Tg | °C | 220 | 235 | 124 |
| Expansion coefficient in the Z direction (Tg or less) | 1/°C | 3.7×10⁻⁵ | 3.6×10⁻⁵ | 5.7×10⁻⁵ |
| Thermal expansion rate in the Z direction (20° to 200°C) | % | 1.14 | 1.06 | 3.15 |
| Z direction (20° to 260°C) | % | 2.41 | 2.33 | >10 |
| Water absorption (after 24 hours' boiling) | % | 1.18 | 1.16 | 1.94 |
| Water absorption (after 48 hours' boiling) | % | 1.29 | 1.24 | 2.18 |
| Resistance of copper foil to peeling | kg/m | 237 | 229 | 210 |
| Thermal resistance to soldering (300°C) | Appearance | Pass | Pass | Swelling |

### Example 6

100 Grams of Sumi ® epoxy ESCN-195XL, (o-cresol novolak type epoxy resin, epoxy equivalent, 197 g/eq; a product of Sumitomo Chemical Co.), 62 g of the imide compound obtained in Example 2, 17 g of a phenol novolak resin, 1 g of 2,4,6-tris(dimethylaminomethyl)phenol, 1 g of carnauba wax, 2 g of a silane coupling agent (SH-6040; a product of Toray Silicone Ltd.) and 427 g of silica were kneaded at
100°C for 5 minutes on a two-roll kneader, cooled and pulverized to produce a molding material. This molding material was press-molded at 170°C for 5 minutes under a pressure of 70 kg/cm and hardened for 5 hours in an oven kept at
180°C. The physical properties of the hardened product obtained are shown in Table 2.

### Comparative example 2

Example 6 was repeated except that 56 g of the phenol novolak resin only was used as a hardener, and that the amount of silica filler was changed from 427 g to 364 g, to obtain a hardened product. The physical properties of this hardened product are shown in Table 2.

**Table 2**

| Example | | Example 6 | Comparative example 2 |
|---|---|---|---|
| Tg | °C | 240 | 170 |
| Thermal expansion coefficient (<Tg) | 1/°C | 2.0×10⁻⁵ | 2.6×10⁻⁵ |
| Thermal expansion rate (20° to 260°C) | % | 1.10 | 1.35 |
| Bending strength ( 20°C) | kg/mm | 14.7 | 14.8 |
| Bending strength (100°C) | kg/mm | 14.0 | 12.1 |
| Bending strength (150°C) | kg/mm | 12.4 | 9.0 |
| Bending strength (180°C) | kg/mm | 9.5 | 2.1 |

It is apparent from Tables 1 and 2 that the composition obtained according to the present invention has excellent thermal resistance, giving molded products having high dimensional stability.

## Claims

1. An imide compound of formula (I) 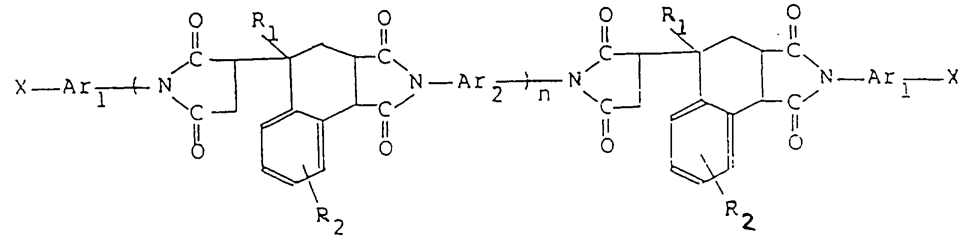 wherein the X's are selected from hydroxyl and amino groups; the Ar₁'s, and the or each Ar₂ if present, are selected independently from aromatic residues; the or each R₁ is selected independently from C₁-C₁₀ alkyl groups; the or each R₂ is selected independently from a hydrogen atom, a hydroxyl group, and C₁-C₂₀ alkyl and alkoxy groups; and n is zero or an integer of up to 30.

2. A compound according to claim 1 wherein each X is amino.

3. A compound according to claim 1 wherein each X is hydroxyl.

4. A method of preparing a compound according to claim 2 which comprises reacting an excess of aromatic diamine with compound of formula (III) 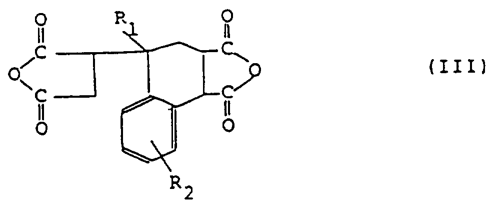 wherein R₁ and R₂ are as defined in claim 1.

5. A method of preparing a compound according to claim 3 which comprises reacting aromatic monoamine having a hydroxyl group and aromatic diamine with compound (III) wherein 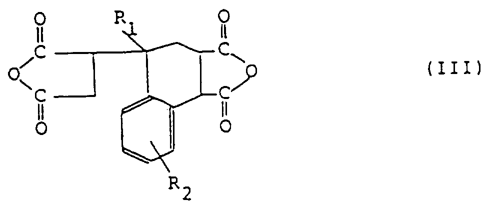 the molar ratio of aromatic diamine to compiund (III) is n to (n + 1), the molar ratio of aromatic monoamine to compound (III) is 2 to (n + 1), and R₁, R₂ and n are as defined in claim 1.

## Patentansprüche

1. Eine Imidverbindung der Formel (I) 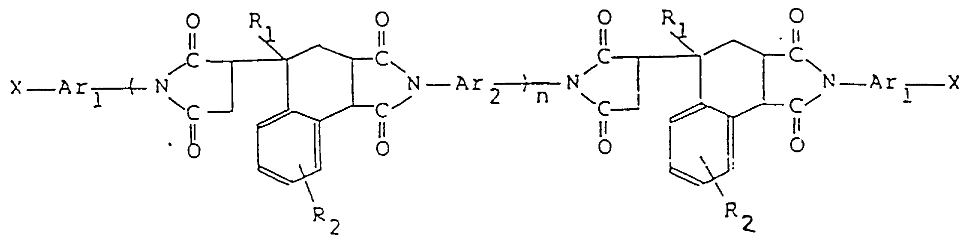 worin die X ausgewählt sind aus Hydroxyl- und Aminogruppen; die Ar₁ und das oder jedes Ar₂, falls vorhanden, unabhängig voneinander ausgewählt sind aus aromatischen Resten, das oder jedes R₁ unabhängig voneinander ausgewählt ist aus C₁-C₁₀-Alkylgruppen, das oder jedes R₂ unabhängig voneinander ausgewählt ist aus einem Wasserstoffatom, einer Hydroxylgruppe und C₁-C₂₀-Alkyl- und Alkoxygruppen und n O oder eine ganze Zahl von bis zu 30 ist.

2. Verbindung nach Anspruch 1, dadurch gekennzeichnet, daß jedes X Amino ist.

3. Verbindung nach Anspruch 1, dadurch gekennzeichnet, daß jedes X Hydroxyl ist.

4. Verfahren zur Herstellung einer Verbindung nach Anspruch 2, dadurch gekennzeichnet, daß man einen Überschuß von aromatischem Diamin mit einer Verbindung der Formel (III) umsetzt 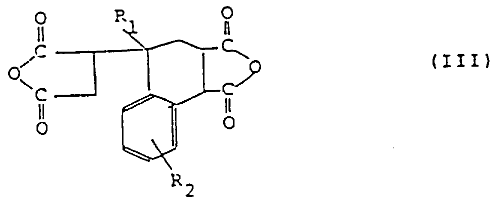 worin R₁ und R₂ wie in Anspruch 1 definiert sind.

5. Verfahren zur Herstellung einer Verbindung nach Anspruch 3, dadurch gekennzeichnet, daß man aromatisches Monoamin mit einer Hydroxylgruppe und aromatisches Diamin mit Verbindung (III) umsetzt 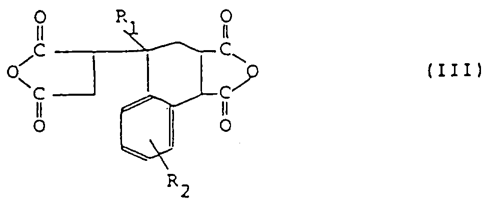 wobei das molare Verhältnis von aromatischem Diamin zur Verbindung (III) n bis (n + 1) ist, das molare Verhältnis von aromatischem Monoamin zur Verbindung (III) 2 bis (n + 1) ist, und R₁, R₂ und n wie in Anspruch 1 definiert sind.

## Revendications

1. Imide de formule (I) 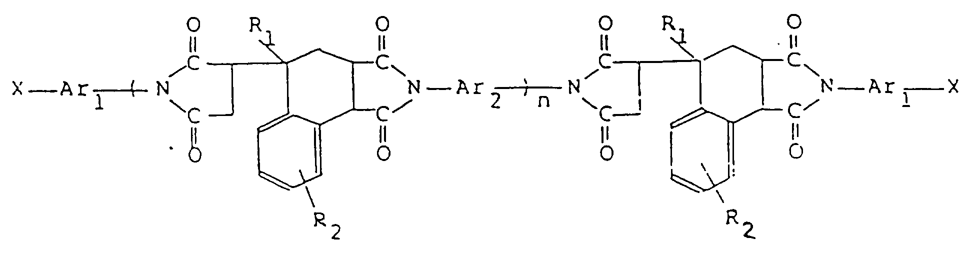 dans lequel les groupes X sont choisis entre des groupes hydroxyle et des groupes amino ; les groupes Ar₁ et le ou chaque groupe Ar₂, s'il est présent, sont choisis, indépendamment, parmi des résidus aromatiques ; le ou chaque groupe R₁ est choisi, indépendamment, parmi des groupes alkyle en C₁ à C₁₀, le ou chaque groupe R₂ est choisi, indépendamment, entre un atome d'hydrogène, un groupe hydroxyle et des groupes alkyle et alkoxy en C₁ à C₂₀ ; et n est égal à zéro ou à un nombre entier allant jusqu'à 30.

2. Composé suivant la revendication 1, dans lequel chaque groupe X est un groupe amino.

3. Composé suivant la revendication 1, dans lequel chaque groupe X est un groupe hydroxyle.

4. Procédé de préparation d'un composé suivant la revendication 2, qui comprend la réaction d'un excès d'une diamine aromatique avec un composé de formule (III) 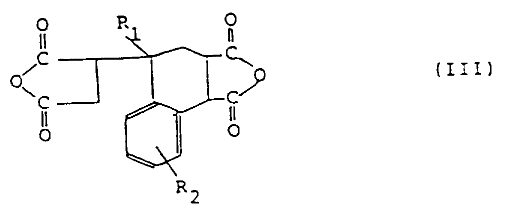 dans laquelle R₁ et R₂ répondent aux définitions suivant la revendication 1.

5. Procédé de préparation d'un composé suivant la revendication 3, qui comprend la réaction d'une mono-amine aromatique ayant un groupe hydroxyle et d'une diamine aromatique avec un composé (III) 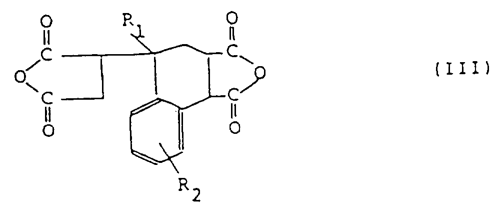 dans lequel le rapport molaire de la diamine aromatique au composé (III) a une valeur de n à (n + 1), le rapport molaire de la mono-amine aromatique au composé (III) a une valeur de 2 à (n + 1), et R₁, R₂ et n répondent aux définitions suivant la revendication 1.
